# FASCICULE DE BREVET EUROPEEN

(11) **EP 4 678 212 B1**
(45) Date de publication et mention de la délivrance du brevet: **02.12.2026**
(21) Numéro de dépôt: 25183425.5
(22) Date de dépôt: 17.06.2025
(51) Int. Cl.: A61M 16/00, A61M 16/01, A61M 16/08, A61M 16/12

(54) **INSTALLATION DE FOURNITURE DE NO COMPRENANT UN APPAREIL DE DÉLIVRANCE DE NO ET UN VENTILATEUR D'ANESTHÉSIE**
INSTALLATION ZUR BEREITSTELLUNG VON NO, BESTEHEND AUS EINEM NO-ABGABEGERÄT UND EINEM ANÄSTHESIEBEATMUNGSGERÄT
INSTALLATION FOR THE SUPPLY OF NO COMPRISING A NO DELIVERY DEVICE AND AN ANESTHESIA VENTILATOR

(30) Priorité: 09.07.2024 FR 2407466
(43) Date de publication de la demande: 14.01.2026
(73) Titulaire: INOSYSTEMS, 92160 Antony (FR)
(72) Inventeur: PROUVEZ, Nathan, 92160 Antony (FR); BLANDIN, Yann, 92160 Antony (FR); THERRIAULT, Patricia, 92160 Antony (FR); MARTINEZ LAFRAYA, Juan, 92160 Antony (FR)
(74) Mandataire: Air Liquide

(56) Documents cités:
- EP-A1- 3 821 929
- WO-A1-2015/153713
- WO-A1-2016/096056
- US-A1- 2023 270 960

## Description

L'invention concerne une installation de fourniture d'un mélange gazeux à base de NO à un patient comprenant un appareil de délivrance de NO pour fournir un mélange gazeux contenant du NO, typiquement un mélange NO/N₂, provenant d'une ou plusieurs sources de NO, telles des bouteilles de gaz sous pression, et un ventilateur d'anesthésie fournissant un gaz à base d'oxygène (i.e. >20%vol. environ).

Le monoxyde d'azote inhalé (NO ou NOi) est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

En général, pour mettre en œuvre une thérapie par NO inhalé, on utilise une installation de fourniture de gaz, aussi appelée installation d'administration de NO, comprenant un appareil de délivrance de NO et un ventilateur médical, c'est-à-dire un appareil d'assistance respiratoire, alimentant un circuit patient.

L'appareil de délivrance de NO permet d'injecter un mélange gazeux à base de NO, typiquement un mélange NO/azote, dans le circuit patient alimenté par ailleurs en un flux gazeux contenant de l'oxygène (au moins environ 20% vol.), tel de l'air ou un mélange oxygène/azote (O₂/N₂), fourni par le ventilateur médical. Cette injection de NO se fait au moyen d'un dispositif injecteur ou module d'injection agencé dans le circuit patient.

Le NO gazeux, typiquement un mélange gazeux NO/azote, alimentant l'appareil de délivrance de NO provient généralement d'un ou plusieurs récipients de gaz sous pression, telles des bouteilles de gaz.

Le circuit patient comprend en général un ou plusieurs conduits flexibles reliés fluidiquement à une interface respiratoire, telle une sonde d'intubation trachéale, un masque respiratoire ou analogue, servant à délivrer au patient à traiter, un mélange gazeux thérapeutique combiné contenant une quantité ou dose donnée de NO, c'est-à-dire une posologie fixée par un médecin.

Afin de pouvoir réguler la quantité de NO fourni, on utilise habituellement un capteur de débit agencé dans le circuit patient, en amont du site d'injection de NO, qui sert à mesurer le débit de gaz contenant de l'oxygène fourni par le ventilateur médical et à fournir cette mesure de débit à l'appareil de délivrance de NO.

Avant administration au patient, le gaz est généralement humidifié au sein d'un humidificateur de gaz agencé dans le circuit patient, entre l'interface respiratoire et le capteur de débit.

Ce type d'installation est utilisé en milieu hospitalier pour administrer le traitement par NO et ainsi soigner les patients ayant besoin d'inhaler du NO pour traiter leur hypertension artérielle pulmonaire, notamment lors d'interventions en chirurgie cardiaque. Des installations de fourniture de NO sont décrites par EP3821929, EP4209243, EP4241817, EP4241812 et EP4295882.

Toutefois, lorsque du NO doit être administré au patient lors d'une intervention de chirurgie, par exemple en chirurgie cardiaque, il peut être nécessaire d'avoir recours à un ventilateur d'anesthésie, aussi appelé station d'anesthésie, permettant de délivrer aussi un gaz anesthésique, tel que de l'isoflurane, le desflurane ou le sevoflurane, utiliser pour sédater/anesthésier le patient, pendant la chirurgie.

Dans ce cas, le circuit patient utilisé est à double branche pour amener le gaz anesthésique jusqu'au patient, via une branche inspiratoire, puis pour récupérer, via une branche expiratoire, les gaz expirés, qui sont enrichis en CO₂ et qui contiennent encore des composés anesthésiques.

Les branches inspiratoire et expiratoire viennent généralement se raccorder au niveau d'une pièce de jonction, aussi appelée pièce en Y, laquelle est par ailleurs reliée à l'interface respiratoire servant à administrer le gaz au patient et à récupérer les gaz expirés, telle qu'une sonde d'intubation trachéale ou un masque respiratoire.

L'appareil de délivrance de NO et le ventilateur d'anesthésie qui est utilisé en lieu et place d'un ventilateur médical classique, doivent alors être raccordés fluidiquement au circuit de gaz servant à administrer le gaz à base de NO au patient.

En particulier, il est nécessaire de raccorder à l'appareil de délivrance de NO non seulement le capteur de débit agencé sur la branche inspiratoire qui sert à mesurer le débit gazeux provenant du ventilateur d'anesthésie afin de permettre de réguler la fourniture de NO (i.e. débit de NO) par l'appareil de délivrance de NO, et le dispositif injecteur de gaz servant à injecter le NO dans la branche inspiratoire mais aussi la ligne de prélèvement de gaz servant à prélever des échantillons gazeux du mélange gazeux à base de NO fourni au patient afin d'opérer un monitorage de la composition de ce mélange et de s'assurer que celui-ci contient les proportions souhaitées de NO et d'oxygène et, à l'inverse, ne contient pas d'espèces toxiques, tel le NO₂, en quantité excessive.

Dans une installation mettant en œuvre un ventilateur classique, le module d'injection de NO et le capteur de débit sont généralement agencés dans la branche inspiratoire en amont de l'humidificateur de gaz et à une distance d'au moins 30 cm à 60 cm de la sortie du ventilateur, alors que la ligne de prélèvement de gaz vient habituellement se raccorder en amont de la pièce en Y, typiquement à environ 15 à 40 cm de celle-ci.

Or, ces raccordements posent problème lorsque le ventilateur n'est pas un ventilateur classique mais un ventilateur d'anesthésie.

En effet, dans ce cas, l'interfaçage avec l'appareil de délivrance de NO, c'est-à-dire le raccordement de la ligne de prélèvement de gaz et du capteur de débit à la branche inspiratoire n'est pas aisé car les installations mettant en œuvre des ventilateurs d'anesthésie utilisent en général un circuit patient plus rudimentaire, à savoir un simple conduit ou tuyau flexible sans humidificateur de gaz car l'humidification se fait directement dans l'appareil au moyen de filtres échangeurs de chaleur et d'humidité embarqués, et avec pièce en Y scellée à la sortie aval de ce conduit ou tuyau flexible. Parfois, la branche expiratoire, qui est généralement aussi formée d'un tuyau flexible, peut être agencée coaxialement dans le tuyau flexible faisant office de branche inspiratoire.

D'autres installations de fourniture de mélanges gazeux contenant du NO connues sont notamment décrites par US2023/270960, WO2016/096056 et WO2015/153713.

Dans tous les cas, on comprend qu'un tel circuit patient rudimentaire ne permet pas une insertion aisée du module de prélèvement de gaz alimentant la ligne de prélèvement de gaz, ni du module d'injection et du capteur de débit, lesquels doivent être reliés à l'appareil de délivrance de NO, sauf desceller la pièce en Y pour insérer le module de prélèvement de gaz et/ou à couper le tuyau flexible pour y insérer le module comprenant le capteur de débit et le module injecteur de NO, ce qui engendre forcément une détérioration du circuit patient et/ou la pièce en Y et, dans tous les cas, est totalement inconcevable, lors d'une intervention chirurgicale.

L'invention vise à résoudre ce problème de raccordement du module de prélèvement de gaz alimentant la ligne de prélèvement de gaz et de celui comprenant le capteur de débit au sein d'un circuit patient rudimentaire associé à un ventilateur d'anesthésie, en particulier utilisé en chirurgie cardiaque.

Une solution selon l'invention concerne alors une installation de fourniture d'un mélange gazeux contenant du NO, typiquement un mélange gazeux NO/N₂, à un patient, i.e. une personne, comprenant :
- un appareil de délivrance de NO configuré pour fournir un flux de gaz contenant du NO, et
- un ventilateur médical pour fournir un flux de gaz respiratoire contenant de l'O₂, à une branche inspiratoire d'un circuit respiratoire,
- un dispositif d'injection de NO, aussi appelé module d'injection, configuré pour injecter le gaz contenant du NO provenant de l'appareil de délivrance de NO dans le flux de gaz respiratoire provenant du ventilateur médical,
- un dispositif de mesure de débit configuré pour mesurer le débit du flux de gaz respiratoire provenant du ventilateur médical, et
- un module de prélèvement de gaz configuré pour prélever, en aval du dispositif d'injection de NO, une partie du gaz circulant dans la branche inspiratoire.

De plus, dans l'installation de fourniture d'un mélange gazeux contenant du NO selon l'invention, le ventilateur médical est un ventilateur d'anesthésie comprenant une sortie de gaz et le dispositif d'injection de NO est agencé en aval du dispositif de mesure de débit.

Par ailleurs, dans l'installation de fourniture d'un mélange gazeux contenant du NO selon l'invention, le dispositif de mesure de débit est raccordé directement à la sortie de gaz du ventilateur d'anesthésie, et le module de prélèvement de gaz est agencé en aval du dispositif d'injection de NO et à une distance (D) d'au moins 30 cm dudit dispositif d'injection de NO.

Selon le mode de réalisation considéré, l'installation de l'invention peut aussi comprendre l'une ou plusieurs des caractéristiques suivantes :
- le dispositif d'injection de NO est agencé entre le dispositif de mesure de débit et le module de prélèvement de gaz.
- le dispositif de mesure de débit est raccordé fluidiquement au dispositif d'injection de NO par l'intermédiaire d'au moins un tronçon de conduit intermédiaire.
- le dispositif d'injection de NO est raccordé directement au dispositif de mesure de débit.
- le dispositif (ou module) d'injection est configuré pour opérer un mélange du gaz contenant du NO provenant de l'appareil de délivrance de NO avec le flux de gaz respiratoire contenant de l'O₂ fourni par le ventilateur médical, et obtenir un mélange gazeux combiné contenant du NO et de l'oxygène, typiquement un mélange gazeux combiné contenant du NO, de l'oxygène et de l'azote.
- plusieurs tronçons de conduit intermédiaires relient fluidiquement le dispositif d'injection de NO au module de prélèvement de gaz.
- le ou les tronçons de conduit intermédiaires comprennent des éléments de tuyaux, de préférence souples ou flexibles, par exemple en polymère.
- il comprend des connecteurs tubulaires pour raccorder le ou les tronçons de conduit intermédiaires, de préférence à raccordement par emboitement.
- des connecteurs tubulaires raccordent fluidiquement ledit au moins un tronçon de conduit intermédiaire au dispositif de mesure de débit et au dispositif d'injection de NO, et/ou plusieurs tronçons de conduit intermédiaires entre eux.
- le module de prélèvement de gaz est agencé en aval du dispositif (ou module) d'injection à une distance comprise entre 30 cm et 80 cm, de préférence à moins de 60 cm, de préférence encore entre 30 et 50 cm.
- le module de prélèvement de gaz est raccordé fluidiquement (directement ou indirectement) à la branche inspiratoire du circuit respiratoire.
- le module de prélèvement de gaz est raccordé fluidiquement à une extrémité amont de la branche inspiratoire du circuit respiratoire, c'est-à-dire à l'entrée de la branche inspiratoire d'un circuit respiratoire rudimentaire, de préférence un circuit respiratoire rudimentaire comprenant une pièce de jonction (i.e. pièce en Y) scellée (i.e. fixée non-démontable) à une extrémité aval de la branche inspiratoire.
- le dispositif d'injection de NO est relié fluidiquement à l'appareil de délivrance de NO de manière à être alimenté en gaz contenant du NO par ledit appareil de délivrance de NO.
- le dispositif de mesure de débit est relié fluidiquement à l'appareil de délivrance de NO de manière à lui fournir des mesures de débit.
- le dispositif de mesure de débit comprend un capteur de débit.
- le capteur de débit est un capteur de débit massique.
- le capteur de débit massique est relié électriquement à l'appareil de délivrance de NO, en particulier aux moyens de pilotage de l'appareil de délivrance de NO.
- le module de prélèvement de gaz est relié fluidiquement à l'appareil de délivrance de NO de manière à lui fournir du gaz circulant dans la branche inspiratoire, en aval du dispositif d'injection de NO.
- le circuit respiratoire comprend en outre une branche expiratoire.
- la branche inspiratoire et la branche expiratoire sont reliées fluidiquement à une pièce de jonction, typiquement une pièce en Y.
- la pièce de jonction est reliée fluidiquement à une interface respiratoire, tel un masque respiratoire, une sonde d'intubation endo-trachéale ou analogue.
- la branche expiratoire est reliée fluidiquement à une entrée de gaz du ventilateur d'anesthésie.
- le circuit respiratoire comprend des conduits ou tuyaux flexibles, en particulier la branche inspiratoire et la branche expiratoire.
- elle comprend en outre au moins un récipient de NO contiennent un mélange NO/N₂, typiquement une ou plusieurs bouteilles de gaz sous pression.
- le (ou les) récipient de NO alimente l'appareil de délivrance de NO en un flux de gaz contenant du NO, typiquement un mélange gazeux formé d'azote et de NO, i.e. un mélange NO/N₂.
- le dispositif d'injection comprend une première entrée de gaz alimentée en flux de gaz respiratoire contenant de l'O₂, i.e. provenant du ventilateur.
- le dispositif d'injection comprend en outre une seconde entrée de gaz alimentée en gaz contenant du NO provenant de l'appareil de délivrance de NO.
- le dispositif d'injection comprend en outre une sortie de gaz fournissant le mélange gazeux combiné contenant du NO et de l'oxygène, obtenu par mélange, au sein du dispositif d'injection, du gaz contenant du NO (e.g. mélange NO/N₂) avec le flux de gaz respiratoire contenant de l'O₂ (e.g. air ou mélange O₂/N₂).
- le dispositif ou module d'injection comprend corps de module comprend un passage de gaz interne (i.e. une chambre ou volume interne) relié fluidiquement à la première entrée de gaz, à la seconde entrée de gaz et à la sortie de gaz.
- le gaz respiratoire provenant du ventilateur pénètre dans le passage de gaz interne du dispositif ou module d'injection par la première entrée de gaz.
- le gaz contenant du NO provenant de l'appareil de délivrance de NO pénètre dans le passage de gaz interne du dispositif ou module d'injection par la seconde entrée de gaz.
- le gaz combiné contenant du NO et de l'oxygène ressort du dispositif ou module d'injection par la sortie de gaz.
- le circuit respiratoire, en particulier la branche inspiratoire, ne comprend aucun humidificateur de gaz, i.e. il est exempt d'humidificateur de gaz.
- le dispositif de mesure de débit, le dispositif d'injection de NO, le module de prélèvement de gaz, ledit au moins un tronçon de conduit intermédiaire et les connecteurs tubulaires forment un ensemble de raccordement indépendant et démontable pour le raccordement d'un appareil de délivrance de NO, configuré pour venir se connecter fluidiquement entre la branche inspiratoire du circuit patient et la sortie de gaz du ventilateur d'anesthésie.

Par ailleurs, selon le mode de réalisation considéré, l'appareil de délivrance de NO peut comprendre l'une ou plusieurs des caractéristiques suivantes :
- il est alimenté en un mélange gazeux formé d'azote et de NO.
- il comprend des moyens de réglage de dose configurés pour permettre à un utilisateur de fixer ou sélectionner une consigne de teneur en NO correspondant à la proportion finale de NO souhaitée dans le mélange gazeux combiné, i.e. une posologie, c'est-à-dire dans le gaz combiné résultant du mélange du flux gazeux contenant le NO (i.e. ) provenant de l'appareil de délivrance de NO avec le flux de gaz respiratoire contenant de l'oxygène provenant du ventilateur.
- les moyens de réglage de dose font partie d'une IHM (interface homme-machine) ou IGU (interface graphique utilisateur).
- les moyens de réglage de dose comprennent une ou des touches tactiles, actionnables par l'utilisateur, s'affichant sur un écran digital à dalle tactile de l'IHM, de préférence du type à affichage en couleurs.
- la consigne de teneur en NO est comprise entre 1 et 80 ppmv, typiquement entre 5 et 40 ppmv.
- il comprend des moyens de mémorisation comprenant une mémoire informatique, telle une mémoire flash, une RAM ou analogue.
- il comprend des moyens de pilotage comprenant au moins un (micro)processeur, tel qu'un microcontrôleur ou analogue.
- les moyens de pilotage comprennent un (ou des) (micro)processeur agencé(s) sur une (ou des) carte électronique.
- les moyens de pilotage comprennent un (ou des) (micro)processeur mettant en œuvre un ou des algorithmes, notamment un (ou des) algorithme de pilotage ou de contrôle des vannes ou valves, de traitement des mesures de débit ou de pression....
- les moyens de mémorisation sont agencés sur la carte électronique.
- il est alimenté électriquement par une ou des sources de courant électrique, typiquement le secteur (110/220V) et/ou une ou des batteries rechargeables.

En outre, selon le mode de réalisation considéré, le ventilateur d'anesthésie peut comprendre l'une ou plusieurs des caractéristiques suivantes :
- il embarque un ou des filtres échangeurs de chaleur et d'humidité permettant d'opérer une humidification du gaz respiratoire directement dans le ventilateur, c'est-à-dire en amont de la sortie de gaz dudit ventilateur d'anesthésie.
- il est configuré pour fournir un flux de gaz respiratoire contenant de l'O₂, de préférence un gaz respiratoire contenant au moins 20%vol. d'oxygène, typiquement au moins 21%vol. d'oxygène environ.
- le flux de gaz respiratoire contenant de l'O₂ sortant du ventilateur d'anesthésie contient de la vapeur d'eau, i.e. il est humidifié.
- de préférence, le flux de gaz respiratoire contenant de l'O₂ sortant du ventilateur d'anesthésie a une température comprise entre 15 et 30 °C.
- le gaz respiratoire contenant de l'O₂ est par exemple de l'air ou un mélange oxygène/azote (O₂/N₂).
- il comprend une soufflante motorisée (i.e. turbine, compresseur ou analogue) délivrant le gaz respiratoire, typiquement de l'air ou un mélange oxygène/azote.
- selon un autre mode de réalisation, il comprend un circuit de gaz interne comprenant une ou des vannes proportionnelles pour acheminer le gaz et contrôler sa fourniture, notamment son débit. Ce circuit est généralement alimenté en gaz respiratoire par une (ou des) prise murale alimentée en gaz par un réseau de canalisations d'un établissement ou bâtiment hospitalier.
- il comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- il est alimenté électriquement par une ou des sources de courant électrique, typiquement le secteur (110/220V) et/ou une ou des batteries rechargeables.

Enfin, selon le mode de réalisation considéré, la ou les récipients de NO alimentant l'installation de l'invention peuvent comprendre l'une ou plusieurs des caractéristiques suivantes :
- le (ou les/chaque) récipient de NO contient un mélange NO/N₂ contenant entre 100 et 2000 ppmv de NO, et de l'azote pour le reste, de préférence un mélange NO/N₂ contenant entre 100 et 1500 ppmv, typiquement entre 200 et 1000 ppmv.
- lorsque les récipients de NO sont pleins, le mélange NO/N₂ y est conditionné à une pression d'au moins 150 bar, de préférence à une pression comprise entre 10 et 250 bar (avant début de soutirage).
- chaque récipient de NO est ou comprend une (ou des) bouteille de gaz ayant une contenance comprise entre 0,5 et 50 L (équivalent en eau).
- chaque récipient de NO est une bouteille de gaz sous pression.
- chaque récipient de NO comprend un corps cylindrique en acier ou en alliage d'aluminium et est équipée d'un robinet simple (sans détendeur) ou à détendeur intégré ou RDI, de préférence un RDI,
- le robinet équipant le/chaque récipient est protégé par un capotage de protection, par exemple en métal ou polymère.

Selon un autre aspect (non-revendiqué), la présente divulgation concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, en particulier dans le cadre d'une intervention chirurgicale avec anesthésie gazeuse, comprenant une administration par inhalation à la personne en ayant besoin, d'un mélange gazeux comprenant de 1 à 80 ppmv de NO et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO assurant une délivrance de NO et un ventilateur d'anesthésie de manière à traiter (au moins partiellement) ladite hypertension pulmonaire et/ou ladite hypoxie, en particulier une hypertension pulmonaire engendrée par une opération de chirurgie cardiaque avec mise du patient sous circulation sanguine extracorporelle (CEC) et anesthésie ventilatoire du patient.

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les pressions sont exprimées en bar absolus, abrévié « bar ».
- les termes « concentration », « quantité », « proportion », « dose » et « teneur » sont considérés comme équivalents et substituables.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de pression », on entend une valeur de pression (e.g. une valeur numérique) ou un signal représentatif d'une telle valeur de pression reflétant ou correspondant à la pression gazeuse mesurée par un capteur de pression ou analogue.
- par « mesure de débit », on entend une valeur de débit (e.g. une valeur numérique) ou un (des) signal (de pression ou de débit) représentatif d'une telle valeur de débit ou permettant de déterminer une telle valeur de débit reflétant ou correspondant au débit gazeux mesurée par un capteur ou analogue.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation d'administration de NO à un patient alimenté par un ventilateur médical classique.
Fig. 2 schématise un mode de réalisation du raccordement du dispositif de mesure de débit, du dispositif d'injection de NO et du module de prélèvement de gaz à la sortie de gaz d'un ventilateur d'anesthésie.

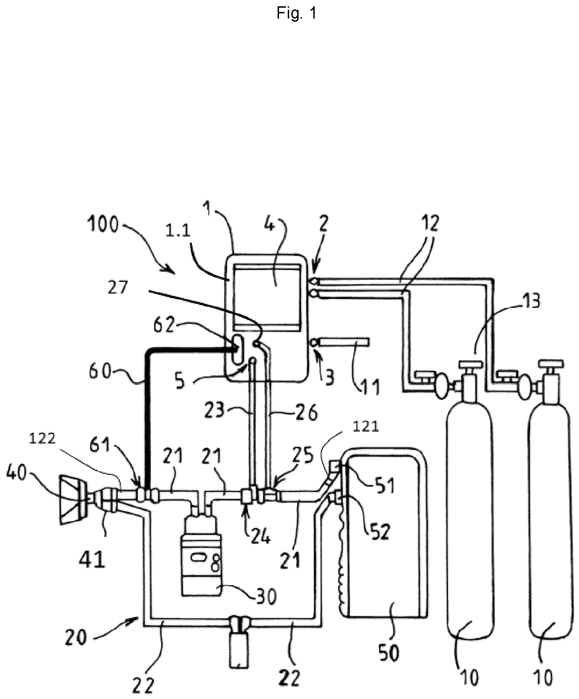

Fig. 1 schématise un mode de réalisation d'une installation d'administration de gaz 100 selon l'invention comprenant un appareil de fourniture de NO 1 fournissant un mélange gazeux à base de monoxyde d'azote (NO), et un ventilateur médical 50 classique, c'est-à-dire qui n'est pas un ventilateur ou une station d'anesthésie, apte à fournir un gaz contenant au moins 20%vol. d'oxygène, tel de l'air ou autre.

L'installation 100 comprend des sources de NO 10, à savoir ici deux récipients ou bouteilles de gaz sous pression contenant chacune un mélange gazeux NO/N₂, à savoir ici un mélange gazeux NO/N₂ contenant entre 100 et 2000 ppmv de NO (reste N₂), typiquement entre 100 et 1000 ppmv de NO (reste N₂), par exemple 450 ou 800 ppm vol. de NO (reste N₂), ou toute autre concentration adéquate, qui alimentent en mélange NO/N₂, le dispositif ou appareil 1 de délivrance ou fourniture de NO permettant de suivre et contrôler la fourniture du mélange gazeux NO/N₂.

Les bouteilles de gaz sont chacune reliées fluidiquement à l'appareil 1 de fourniture de NO, via des lignes d'amenée de gaz 12, tel que des tuyaux ou conduits flexibles ou analogues équipés de connecteurs de raccordement, qui peuvent être en outre équipées de dispositifs de régulation et/ou de suivi de la pression de gaz, tels que détendeur de gaz 13, manomètres...

Les lignes d'amenée de gaz 12 sont raccordées fluidiquement à des entrées de gaz 2 de l'appareil de délivrance de NO 1 qui alimentent un circuit de gaz interne (non montré), servant à acheminer le gaz au sein de l'appareil de fourniture de NO 1, c'est-à-dire dans le boitier ou carcasse externe de l'appareil 1. Un tel circuit de gaz interne comprend un ou des tronçons de circuit reliés fluidiquement aux entrées 2 de NO qui sont alimentées en mélange NO/N₂ provenant des bouteilles de gaz 10.

Le circuit de gaz comprend classiquement des électrovannes ou analogues qui sont pilotées par les moyens de pilotage de l'appareil 1 pour contrôler le flux de gaz au sein du circuit de gaz interne, ainsi que d'autres composants, tel qu'un ou plusieurs capteurs de pression ou de débit, par exemple un contrôleur de débit massique ou MFC (i.e. Mass Flow Controller).

L'appareil de délivrance de NO 1 comprend par ailleurs une entrée d'oxygène 3 reliée fluidiquement, via une ligne d'amenée d'oxygène 11, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier. Ceci permet d'alimenter le circuit de gaz interne 200 en oxygène quand cela est nécessaire.

Sur Fig. 1, le ventilateur médical 50 est un appareil d'assistance respiratoire classique, fournissant un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote (N₂/O₂), mais ne permettant pas d'opérer une anesthésie du patient.

Le ventilateur médical 50 et l'appareil de fourniture de NO 1 de l'installation 100 sont en communication fluidique avec un circuit respiratoire 20, aussi appelé circuit patient qui comprend une branche inspiratoire 21 et une branche expiratoire 22.

La branche inspiratoire 2 sert à acheminer le flux gazeux vers l'interface respiratoire 40 pour fournir le flux gazeux thérapeutique au patient, c'est-à-dire un mélange gazeux combiné contenant la posologie en NO souhaitée.

Le mélange gazeux combiné à administrer au patient est formé par mélange du flux à base d'oxygène (e.g. air ou mélange O₂/N₂) provenant du ventilateur 50 et du flux contenant le NO, i.e. le mélange gazeux NO/N₂, délivré par l'appareil de délivrance de NO 1.

Pour ce faire, l'appareil de délivrance de NO 1 fournit ou injecte le mélange NO/N₂ dans la branche inspiratoire 21, via un conduit ou une ligne d'injection 23, reliant fluidiquement le circuit de gaz interne de l'appareil de fourniture de NO 1 à un dispositif ou module d'injection 24 agencé sur la branche inspiratoire 21. Le dispositif d'injection 24 est configuré pour opérer un mélange du gaz contenant du NO avec le flux de gaz respiratoire contenant de l'O₂ provenant du ventilateur 50 et obtenir ainsi le mélange gazeux combiné contenant du NO et de l'oxygène, c'est-à-dire le mélange gazeux final administré au patient.

Le dispositif d'injection 24 comprend une première entrée de gaz alimentée en flux de gaz respiratoire contenant de l'O₂ provenant du ventilateur médical 50, une seconde entrée de gaz alimentée en gaz contenant du NO, i.e. provenant de l'appareil de délivrance de NO 1, et une sortie de gaz fournissant le mélange gazeux combiné contenant du NO et de l'oxygène, obtenu par mélange, au sein du dispositif d'injection 24, du gaz contenant du NO avec le flux de gaz respiratoire contenant de l'O₂. Le flux de NO/N₂ amené par la ligne d'injection 23 se mélange alors, dans un passage ou une chambre interne du dispositif d'injection 24, au flux de gaz à base d'oxygène (> 20% d'O₂), e.g. de l'air ou un mélange oxygène/azote, délivré par le ventilateur 50 et véhiculé par la branche inspiratoire 21 de sorte d'obtenir le mélange combiné désiré à administrer au patient contenant essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux final NO/N₂/O₂ .

Comme visible en Fig. 1, lorsqu'une telle installation 100 est alimentée par un ventilateur médical 50 classique, la branche inspiratoire 21 du circuit 20 comprend aussi un humidificateur de gaz 30 agencé en aval du dispositif d'injection 24. Il permet d'humidifier le flux de gaz combiné, e.g. le mélange NO/N₂/O₂, avant qu'il ne soit administré par inhalation au patient à traiter, au moyen d'une interface respiratoire 40, telle une sonde d'intubation trachéale, un masque respiratoire ou analogue.

Par ailleurs, le circuit patient 20 comprend aussi une branche expiratoire 22 permettant une récupération des gaz expirés par le patient. La branche inspiratoire 21 (via on extrémité aval 122) et la branche expiratoire 22 sont reliées fluidiquement à une pièce de jonction 41, telle une pièce en Y, laquelle est également raccordée à l'interface respiratoire 40 afin d'assurer les échanges gazeux en direction ou provenant des poumons du patient.

Dans cette installation classique 100, la branche inspiratoire 21 est raccordée fluidiquement, via son extrémité amont 121, à un port de sortie 51 du ventilateur médical 50, tel un connecteur, raccord ou analogue, de manière à récupérer et acheminer le gaz à base d'oxygène, typiquement de l'air ou mélange N₂/O₂ fourni par le ventilateur médical 50, alors que la branche expiratoire 22 est raccordée fluidiquement à un port d'entrée 52 du ventilateur médical 50, tel un connecteur, raccord ou analogue, de manière à retourner au ventilateur médical 50 tout ou partie du débit des gaz expirés par le patient (i.e. gaz enrichis en CO₂).

Afin de pouvoir mesurer le débit de gaz délivré par le ventilateur 50, tel de l'air ou N₂/O₂, circulant dans la branche inspiratoire 21, en amont du dispositif d'injection 24, est prévu un dispositif de mesure de débit 25, typiquement un capteur de débit, par exemple du type à fil chaud, à différentiel de pression ou autre. Le capteur de débit 25 est relié à l'appareil de délivrance de NO 1, via une (des) ligne de mesure de débit 26 venant se raccorder à un port de connexion 27 de l'appareil 1.

Connaître le débit de gaz respiratoire permettent de contrôler ou réguler plus efficacement la délivrance du flux de NO (i.e. N₂/O₂) par l'appareil de délivrance de NO 1, en particulier le débit de NO, puisque les mesures de débit opérées par le capteur de débit 25 sont retournées aux moyens de pilotage à (micro)processeur de l'appareil de délivrance de NO 1, typiquement un (micro)contrôleur, qui traitent ces mesures de débit pour déterminer le flux de NO à fournir, en fonction de la posologie de NO désirée et de la proportion de NO dans le flux de NO/N₂ provenant des bouteilles de gaz 10.

Classiquement, les moyens de pilotage de l'appareil 1 (non montrés car agencés dans la carcasse de l'appareil 1), tel un (micro)contrôleur, comprennent une (des) carte électronique comprenant un (ou plusieurs) microprocesseur(s) mettant en œuvre un ou des algorithmes. Ils permettent notamment d'ajuster ou contrôler le débit de gaz à base de NO en pilotant tout ou partie des (électro)vannes, et en outre d'opérer des calculs et/ou de contrôler ou commander tous les éléments électromécaniques de l'appareil 1, tels que les capteurs, les électrovannes, les affichages ...

Habituellement, l'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

L'alimentation électrique de l'appareil de fourniture de NO 1, en particulier des composants nécessitant du courant électrique pour fonctionner, tels les moyens de pilotage, l'afficheur graphique..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

En outre, l'installation 100 comprend aussi une ligne de prélèvement de gaz 60 qui relie fluidiquement la branche inspiratoire 21 à un port d'entrée 62 de l'appareil de fourniture de NO 1. La ligne de prélèvement de gaz 60, tel un tuyau flexible ou analogue, vient se raccorder à un module de prélèvement 61 inséré dans la branche inspiratoire 21, entre l'humidificateur 30 et la pièce de jonction 41, i.e. pièce en Y, typiquement à proximité immédiate de la pièce de jonction 41. Le module de prélèvement 61 et la ligne de prélèvement de gaz 60 permettent de prélever des échantillons de gaz combiné et de les convoyer jusqu'au dispositif de fourniture de NO 1 où ils sont analysés dans un analyseur de gaz interne (non montré) comprenant des capteurs de NO₂, de NO et d'O₂, typiquement des cellules électrochimiques, reliés électriquement aux moyens de pilotage. Ceci permet d'opérer un monitorage du gaz et de vérifier sa conformité.

En particulier, il convient de vérifier que la composition du gaz combiné est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique, qu'il ne contient pas une teneur en NO₂ trop élevée et que sa teneur en NO correspond à la posologie souhaitée, i.e. dose de NO à administrer par inhalation qui est habituellement choisie par le personnel soignant, i.e. médecin ou analogue. Les moyens de pilotage de l'appareil 1 traitent les signaux provenant des capteurs de l'analyseur de gaz et déclenchent des alarmes en cas de détermination de proportions non-conformes. Les valeurs de teneurs en NO, NO2 et O₂ sont préférentiellement affichées sur l'afficheur graphique 4 de l'IHM.

Dans une telle installation 1, le module d'injection de NO 24 et le dispositif de mesure de débit ou capteur de débit 25 sont généralement agencés dans la branche inspiratoire 21 en amont de l'humidificateur de gaz 30 et à une distance d'au moins 30 cm à 60 cm de la sortie du ventilateur 50, alors que la ligne de prélèvement de gaz 23 vient se raccorder en amont de la pièce en Y 41, typiquement à environ 15 à 40 cm de celle-ci.

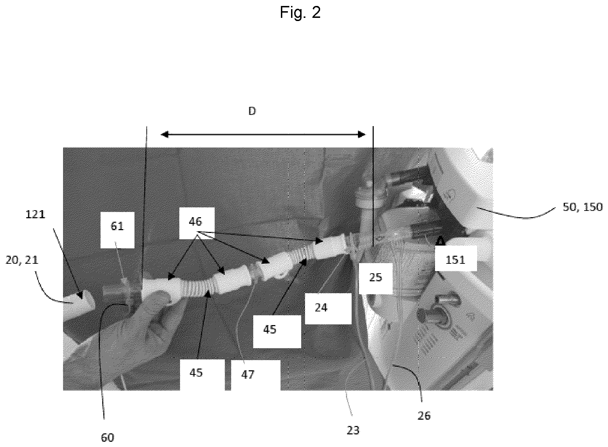

Or, comme déjà expliqué, lorsque le ventilateur 50 est, comme illustré en Fig. 2, un ventilateur ou une station d'anesthésie 150 du type de ceux utilisés pendant les interventions chirurgicales, notamment en chirurgie cardiaque avec CEC, et que le circuit patient 20 est rudimentaire, notamment avec pièce en Y 41 scellée (i.e. non-démontable) à l'extrémité aval 122 de la branche inspirateur 21, opérer un raccordement, à la fin de l'intervention chirurgicale, de l'appareil de délivrance de NO 1 à la branche inspiratoire 1, pour fournir du NO au patient et éviter ou minimiser ainsi le phénomène d'hypertension pulmonaire pouvant résulter de la CEC notamment, n'est pas aisé, voire impossible.

Dès lors, dans le cadre de l'invention, on propose un arrangement ou aménagement particulier venant s'insérer entre le circuit patient 20, typiquement la branche inspiratoire 21, et la sortie 151 du ventilateur d'anesthésie 150.

Plus précisément, comme illustré en Fig. 2, selon l'invention, le dispositif ou module de mesure de débit 25 est raccordé directement à la sortie de gaz 151 du ventilateur d'anesthésie 150 et non plus raccordé à plus de 30 cm à 60 cm comme pour un ventilateur classique 50 tel que schématise en Fig. 1.

De plus, le module de prélèvement de gaz 61 est agencé, quant à lui, en aval du dispositif ou module de mesure de débit 25 et à une distance D d'au moins 30 cm du dispositif ou module de mesure de débit 25, en étant raccordé fluidiquement l'un à l'autre au moyen d'un ou plusieurs tronçons de conduit intermédiaires 45, par exemple deux tronçons de conduit intermédiaires 45, comme visible sur Fig. 2, et de connecteurs tubulaires 46.

Il peut être aussi nécessaire ou souhaitable d'utiliser un ou des éléments de raccordement fluidique 47 additionnels pour raccorder par exemple deux connecteurs tubulaires 46 l'un avec l'autre, comme schématisé en Fig. 2. De tels connecteurs tubulaires 46 et éléments de raccordement fluidique 47 additionnels sont classiques et sont par exemple en polymère.

Les tronçons de conduit intermédiaires 45 préférentiellement des tuyaux souples ou flexibles, par exemple en polymère.

Par ailleurs, le dispositif d'injection 24 de NO vient se raccorder entre le dispositif ou module de mesure de débit 25 et le module de prélèvement de gaz 61.

Dans le mode de réalisation de Fig. 2, le dispositif d'injection 24 de NO est fixé directement au dispositif ou module de mesure de débit 25, par exemple emboités l'un dans l'autre. Les tronçons de conduit intermédiaires 45 permettent donc de relier la sortie du dispositif d'injection 24 de NO à l'entrée du module de prélèvement de gaz 61.

Le module de prélèvement de gaz 61 qui est agencé en aval dispositif d'injection 24 de NO, est situé à une distance D comprise entre 30 cm et 80 cm, de préférence 30 cm et 50 cm dudit dispositif d'injection 24 de NO, ce qui permet de laisser suffisamment de temps pour que le mélange du flux de NO/N₂ et du flux de gaz respiratoire (e.g. air ou O₂/N₂) s'homogénéise et que les échantillons de gaz prélevés par le module de prélèvement de gaz 61 soient représentatifs des proportions de NO, O₂ et NO₂ éventuels dans le mélange combiné obtenu après mélange desdits flux.

Comme expliqué ci-avant, le dispositif de mesure de débit 25 vient se raccorder à l'appareil de délivrance de NO 1, i.e. au port de connexion 27 de l'appareil 1, via la ligne de mesure de débit 26 servant à transmettre les mesures de débit opérées par le dispositif de mesure de débit 25. Le dispositif de mesure de débit 25 est préférentiellement un capteur de débit massique et relié électriquement à l'appareil de délivrance de NO 1.

De façon analogue, le dispositif d'injection 24 de NO est relié, via un conduit ou une ligne d'injection 23, au port de sortie 5 de l'appareil de délivrance de NO 1 afin de permettre de convoyer puis d'injecte le mélange NO/N₂ dans le flux de gaz respiratoire provenant du ventilateur d'anesthésie 150.

De même, le module de prélèvement de gaz 61 est, quant à lui, relié, via la ligne de prélèvement de gaz 60, au port 62 de l'appareil de délivrance de NO 1 afin d'y acheminer les échantillons gazeux à tester.

Selon un mode de réalisation, le dispositif ou module d'injection 24 comprend un corps de module comprend un passage de gaz interne, i.e. une chambre ou volume interne, communiquant fluidiquement avec une première entrée de gaz alimentée en flux de gaz respiratoire contenant de l'O₂, i.e. provenant du ventilateur 150 ; une seconde entrée de gaz alimentée en gaz contenant du NO provenant de l'appareil de délivrance de NO 1 ; et une sortie de gaz fournissant le mélange gazeux combiné contenant du NO et de l'oxygène, obtenu par mélange, au sein du passage de gaz interne du dispositif d'injection, du gaz contenant du NO (e.g. mélange NO/N₂) avec le flux de gaz respiratoire contenant de l'O₂ (e.g. air ou mélange O₂/N₂).

Selon un mode de réalisation, le dispositif de mesure de débit 25 comprend un corps principal traversé par un passage de gaz interne comprenant un orifice d'entrée par lequel le flux de gaz respiratoire contenant de l'O₂, i.e. provenant du ventilateur 150 peut pénétrer dans le passage interne, et un orifice de sortie par lequel ledit flux de gaz respiratoire ressort du passage interne. Il comprend en outre une carte électronique faisant office de capteur de débit massique portée par le corps principal et en communication fluidique avec le passage interne pour y opérer des mesures de débit gazeux.

Selon un mode de réalisation, le module de prélèvement de gaz 61 comprend lui aussi un corps principal traversé par un passage de gaz interne comprenant un orifice d'entrée par lequel le flux de gaz combiné (i.e. mélange NO/N₂/O₂) peut pénétrer dans le passage interne, et un orifice de sortie par lequel ledit flux de gaz combiné ressort du passage interne et est ensuite convoyé par la branche inspiratoire 21 du circuit patient 20 et ce, jusqu'au patient. Il comprend en outre un connecteur ou analogue pour le raccordement fluidique de la ligne de prélèvement de gaz 62 de manière à permettre de prélever des échantillons gazeux de mélange combiné au sein dudit passage interne, lesquels sont acheminés ensuite par la ligne de prélèvement de gaz 62 jusqu'à l'appareil 1 où ils sont analysés.

Afin de faciliter leur raccordement fluidique, le dispositif de mesure de débit 25, le dispositif d'injection 24 de NO et le module de prélèvement de gaz 61 sont préférentiellement équipés d'embouts tubulaires, aussi appelés raccords ou connecteurs, permettant leur raccordement mécanique et fluidique notamment, selon le cas, à la sortie 151 du ventilateur d'anesthésie 150, au(x) tronçon(s) de conduit intermédiaire(s) 45 et à l'extrémité amont 121 de la branche inspiratoire 21, en particulier un raccordement par insertion à force, emboitement ou analogue.

Par exemple, la première entrée de gaz et la sortie de gaz du dispositif ou module d'injection 24, l'orifice d'entrée et l'orifice de sortie du dispositif de mesure de débit 25 et ceux du module de prélèvement de gaz 61 peuvent être agencés dans, i.e. portés par, de tels embouts tubulaires.

D'une façon générale, le dispositif de mesure de débit 25, le dispositif d'injection 24 de NO, le module de prélèvement de gaz 61, ledit au moins un tronçon de conduit intermédiaire 45 et les connecteurs tubulaires 46 forment un ensemble de raccordement indépendant et démontable pour le raccordement de l'appareil de délivrance de NO 1, lequel vient se connecter ou raccorder fluidiquement entre la branche inspiratoire 21 du circuit patient 20 et la sortie de gaz 151 du ventilateur d'anesthésie 150, comme schématisé en Fig. 2, et ce, même lorsque le circuit patient 20 est rudimentaire, par exemple formé d'un simple conduit de gaz, et y compris lorsque ce circuit 30 comprend une pièce en Y scellée à l'extrémité aval de sa branche inspiratoire 21, c'est-à-dire non prévue pour être facilement dissociable ou démontable de la branche inspiratoire 21.

Une installation d'administration de gaz 100 selon l'invention peut être utilisée pour administrer par inhalation du monoxyde d'azote (NO), i.e. le mélange final obtenu NO/O₂/N₂, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter les hypertensions pulmonaires (HP) en chirurgie cardiaque chez l'adulte ou l'enfant.

## Revendications

1. Installation (100) de fourniture d'un mélange gazeux contenant du NO à un patient comprenant :
- un appareil de délivrance de NO (1) configuré pour fournir un flux de gaz contenant du NO, et
- un ventilateur médical (50) pour fournir un flux de gaz respiratoire contenant de l'O₂, à une branche inspiratoire (21) d'un circuit respiratoire (20), ledit ventilateur médical (50) étant un ventilateur d'anesthésie (150) comprenant une sortie de gaz (151),
- un dispositif d'injection (24) de NO configuré pour injecter le gaz contenant du NO provenant de l'appareil de délivrance de NO (1) dans le flux de gaz respiratoire provenant du ventilateur médical (50),
- un dispositif de mesure de débit (25) configuré pour mesurer le débit du flux de gaz respiratoire provenant du ventilateur médical (50), le dispositif d'injection (24) de NO étant agencé en aval du dispositif de mesure de débit (25), et
- un module de prélèvement de gaz (61) configuré pour prélever, en aval du dispositif d'injection (24) de NO, une partie du gaz circulant dans la branche inspiratoire (21),
**caractérisée en ce que** le dispositif de mesure de débit (25) est raccordé directement à la sortie de gaz (151) du ventilateur d'anesthésie (150), et le module de prélèvement de gaz (61) est agencé en aval du dispositif d'injection (24) de NO, à une distance (D) d'au moins 30 cm dudit dispositif d'injection (24) de NO.

2. Installation selon la revendication 1, **caractérisée en ce que** le dispositif d'injection (24) de NO est raccordé directement au dispositif de mesure de débit (25).

3. Installation selon les revendications 1 et 2, **caractérisée en ce que** le dispositif de mesure de débit (25) est raccordé fluidiquement au dispositif d'injection (24) de NO par l'intermédiaire d'au moins un tronçon de conduit intermédiaire (45).

4. Installation selon la revendication 3, **caractérisée en ce que** plusieurs tronçons de conduit intermédiaires (45) relient fluidiquement le dispositif d'injection (24) de NO au module de prélèvement de gaz (61).

5. Installation selon l'une des revendications 3 ou 4, **caractérisée en ce que** des connecteurs tubulaires (46) raccordent fluidiquement ledit au moins un tronçon de conduit intermédiaire (45) au dispositif de mesure de débit (25) et au dispositif d'injection (24) de NO, et/ou plusieurs tronçons de conduit intermédiaires (45) entre eux.

6. Installation selon la revendication 1, **caractérisée en ce que** le module de prélèvement de gaz (61) est agencé en aval du dispositif d'injection (24) de NO et à une distance (D) comprise entre 30 cm et 80 cm.

7. Installation selon la revendication 1, **caractérisée en ce que** le module de prélèvement de gaz (61) est raccordé fluidiquement à la branche inspiratoire (21) du circuit respiratoire (20).

8. Installation selon la revendication 4, **caractérisée en ce que** le module de prélèvement de gaz (61) est raccordé fluidiquement à une extrémité amont (121) de la branche inspiratoire (21).

9. Installation selon les revendications 1 et 4, **caractérisée en ce que** le circuit respiratoire (20) comprend une pièce de jonction (41) scellée à une extrémité aval (122) de la branche inspiratoire (21), de préférence la pièce de jonction (41) est une pièce en Y.

10. Installation selon les revendications 3 et 5, **caractérisée en ce que** le dispositif de mesure de débit (25), le dispositif d'injection (24) de NO, le module de prélèvement de gaz (61), ledit au moins un tronçon de conduit intermédiaire (45) et les connecteurs tubulaires (46) forment un ensemble de raccordement indépendant et démontable pour le raccordement de l'appareil de délivrance de NO (1), configuré pour venir se connecter fluidiquement entre la branche inspiratoire (21) du circuit patient (20) et la sortie de gaz (151) du ventilateur d'anesthésie (150).

11. Installation selon l'une des revendications 1 ou 6, **caractérisée en ce que** le module de prélèvement de gaz (61) est agencé en aval du dispositif d'injection (24) de NO et à une distance (D) de moins de 60 cm.

12. Installation selon l'une des revendications 1, 6 ou 11 **caractérisée en ce que** le module de prélèvement de gaz (61) est agencé en aval du dispositif d'injection (24) de NO et à une distance (D) comprise entre 30 cm et 50 cm.

13. Installation selon la revendication 1, **caractérisée en ce que** le module de prélèvement de gaz (61) est raccordé fluidiquement à une entrée de la branche inspiratoire (21) du circuit respiratoire (20), ledit circuit respiratoire (20) étant un circuit rudimentaire comprenant une pièce de jonction (41) scellée située à une extrémité aval de la branche inspiratoire (21).

14. Installation selon la revendication 1, **caractérisée en ce que** :
- le dispositif d'injection de NO (24) est relié fluidiquement à l'appareil de délivrance de NO (1) de manière à être alimenté en gaz contenant du NO par ledit appareil de délivrance de NO (1) et/ou
- le dispositif de mesure de débit (25) comprend un capteur de débit relié fluidiquement à l'appareil de délivrance de NO (1) de manière à lui fournir des mesures de débit, de préférence le capteur de débit est un capteur de débit massique

15. Installation selon la revendication 1, **caractérisée en ce que** le module de prélèvement de gaz (61) est relié fluidiquement à l'appareil de délivrance de NO (1) de manière à lui fournir du gaz circulant dans la branche inspiratoire (21), en aval du dispositif d'injection de NO (24).

## Patentansprüche

1. Eine Anlage (100) zur Zufuhr eines NO-haltigen Gasgemisches zu einem Patienten, umfassend:
- ein NO-Verabreichungsgerät (1), das konfiguriert ist, um einen Strom von NO-haltigem Gas bereitzustellen, und
- ein medizinisches Beatmungsgerät (50) zur Zufuhr eines O2-haltigen Atemgasstroms zu einem inspiratorischen Zweig (21) eines Atemkreislaufs (20), wobei das besagte medizinische Beatmungsgerät (50) ein Anästhesiebeatmungsgerät (150) ist, das einen Gasauslass (151) umfasst,
- eine NO-Injektionsvorrichtung (24), die konfiguriert ist, um das vom NO-Verabreichungsgerät (1) stammende NO-haltige Gas in den vom medizinischen Beatmungsgerät (50) stammenden Atemgasstrom zu injizieren,
- eine Durchflussmessvorrichtung (25), die konfiguriert ist, um den Durchfluss des vom medizinischen Beatmungsgerät (50) stammenden Atemgasstroms zu messen, wobei die NO-Injektionsvorrichtung (24) stromabwärts der Durchflussmessvorrichtung (25) angeordnet ist, und
- ein Gasentnahmemodul (61), das konfiguriert ist, um stromabwärts der NO-Injektionsvorrichtung (24) einen Teil des im inspiratorischen Zweig (21) zirkulierenden Gases zu entnehmen,
**dadurch gekennzeichnet, dass** die Durchflussmessvorrichtung (25) direkt mit dem Gasauslass (151) des Anästhesiebeatmungsgeräts (150) verbunden ist, und das Gasentnahmemodul (61) stromabwärts der NO-Injektionsvorrichtung (24) in einem Abstand (D) von mindestens 30 cm von der besagten NO-Injektionsvorrichtung (24) angeordnet ist.

2. Die Anlage nach Anspruch 1, **dadurch gekennzeichnet, dass** die NO-Injektionsvorrichtung (24) direkt mit der Durchflussmessvorrichtung (25) verbunden ist.

3. Die Anlage nach den Ansprüchen 1 und 2, **dadurch gekennzeichnet, dass** die Durchflussmessvorrichtung (25) fluidisch mit der NO-Injektionsvorrichtung (24) über mindestens einen Abschnitt einer Zwischenleitung (45) verbunden ist.

4. Die Anlage nach Anspruch 3, **dadurch gekennzeichnet, dass** mehrere Abschnitte von Zwischenleitungen (45) die NO-Injektionsvorrichtung (24) fluidisch mit dem Gasentnahmemodul (61) verbinden.

5. Die Anlage nach einem der Ansprüche 3 oder 4, **dadurch gekennzeichnet, dass** rohrförmige Verbinder (46) den besagten mindestens einen Abschnitt der Zwischenleitung (45) fluidisch mit der Durchflussmessvorrichtung (25) und der NO-Injektionsvorrichtung (24) verbinden, und/oder mehrere Abschnitte von Zwischenleitungen (45) untereinander verbinden.

6. Die Anlage nach Anspruch 1, **dadurch gekennzeichnet, dass** das Gasentnahmemodul (61) stromabwärts der NO-Injektionsvorrichtung (24) und in einem Abstand (D) zwischen 30 cm und 80 cm angeordnet ist.

7. Die Anlage nach Anspruch 1, **dadurch gekennzeichnet, dass** das Gasentnahmemodul (61) fluidisch mit dem inspiratorischen Zweig (21) des Atemkreislaufs (20) verbunden ist.

8. Die Anlage nach Anspruch 4, **dadurch gekennzeichnet, dass** das Gasentnahmemodul (61) fluidisch mit einem stromaufwärtigen Ende (121) des inspiratorischen Zweigs (21) verbunden ist.

9. Die Anlage nach den Ansprüchen 1 und 4, **dadurch gekennzeichnet, dass** der Atemkreislauf (20) ein Verbindungsstück (41) umfasst, das an einem stromabwärtigen Ende (122) des inspiratorischen Zweigs (21) versiegelt ist, vorzugsweise ist das Verbindungsstück (41) ein Y-Stück.

10. Die Anlage nach den Ansprüchen 3 und 5, **dadurch gekennzeichnet, dass** die Durchflussmessvorrichtung (25), die NO-Injektionsvorrichtung (24), das Gasentnahmemodul (61), der besagte mindestens eine Abschnitt der Zwischenleitung (45) und die rohrförmigen Verbinder (46) eine unabhängige und demontierbare Verbindungseinheit für die Verbindung des NO-Verabreichungsgeräts (1) bilden, die konfiguriert ist, um fluidisch zwischen dem inspiratorischen Zweig (21) des Patientenkreislaufs (20) und dem Gasauslass (151) des Anästhesiebeatmungsgeräts (150) verbunden zu werden.

11. Die Anlage nach einem der Ansprüche 1 oder 6, **dadurch gekennzeichnet, dass** das Gasentnahmemodul (61) stromabwärts der NO-Injektionsvorrichtung (24) und in einem Abstand (D) von weniger als 60 cm angeordnet ist.

12. Die Anlage nach einem der Ansprüche 1, 6 oder 11, **dadurch gekennzeichnet, dass** das Gasentnahmemodul (61) stromabwärts der NO-Injektionsvorrichtung (24) und in einem Abstand (D) zwischen 30 cm und 50 cm angeordnet ist.

13. Die Anlage nach Anspruch 1, **dadurch gekennzeichnet, dass** das Gasentnahmemodul (61) fluidisch mit einem Eingang des inspiratorischen Zweigs (21) des Atemkreislaufs (20) verbunden ist, wobei der besagte Atemkreislauf (20) ein rudimentärer Kreislauf ist, der ein Verbindungsstück (41) umfasst, das versiegelt an einem stromabwärtigen Ende des inspiratorischen Zweigs (21) angeordnet ist.

14. Die Anlage nach Anspruch 1, **dadurch gekennzeichnet, dass**:
- die NO-Injektionsvorrichtung (24) fluidisch mit dem NO-Verabreichungsgerät (1) verbunden ist, um mit NO-haltigem Gas durch das besagte NO-Verabreichungsgerät (1) versorgt zu werden und/oder
- die Durchflussmessvorrichtung (25) einen Durchflusssensor umfasst, der fluidisch mit dem NO-Verabreichungsgerät (1) verbunden ist, um ihm Durchflussmessungen zu liefern, vorzugsweise ist der Durchflusssensor ein Massendurchflusssensor

15. Die Anlage nach Anspruch 1, **dadurch gekennzeichnet, dass** das Gasentnahmemodul (61) fluidisch mit dem NO-Verabreichungsgerät (1) verbunden ist, um ihm Gas zuzuführen, das im inspiratorischen Zweig (21) stromabwärts der NO-Injektionsvorrichtung (24) zirkuliert.

## Claims

1. An installation (100) for supplying a gaseous mixture containing NO to a patient comprising:
- a NO delivery apparatus (1) configured to supply a flow of gas containing NO, and
- a medical ventilator (50) for supplying a flow of respiratory gas containing O2, to an inspiratory branch (21) of a respiratory circuit (20), said medical ventilator (50) being an anesthesia ventilator (150) comprising a gas outlet (151),
- a NO injection device (24) configured to inject the gas containing NO originating from the NO delivery apparatus (1) into the flow of respiratory gas originating from the medical ventilator (50),
- a flow rate measurement device (25) configured to measure the flow rate of the respiratory gas originating from the medical ventilator (50), the NO injection device (24) being arranged downstream of the flow rate measurement device (25), and
- a gas sampling module (61) configured to sample, downstream of the NO injection device (24), a portion of the gas circulating in the inspiratory branch (21),
**characterized in that** the flow rate measurement device (25) is directly connected to the gas outlet (151) of the anesthesia ventilator (150), and the gas sampling module (61) is arranged downstream of the NO injection device (24), at a distance (D) of at least 30 cm from said NO injection device (24).

2. The installation according to claim 1, **characterized in that** the NO injection device (24) is directly connected to the flow rate measurement device (25).

3. The installation according to claims 1 and 2, **characterized in that** the flow rate measurement device (25) is fluidically connected to the NO injection device (24) via at least one section of intermediate conduit (45).

4. The installation according to claim 3, **characterized in that** several sections of intermediate conduit (45) fluidically connect the NO injection device (24) to the gas sampling module (61).

5. The installation according to one of claims 3 or 4, **characterized in that** tubular connectors (46) fluidically connect said at least one section of intermediate conduit (45) to the flow rate measurement device (25) and to the NO injection device (24), and/or several sections of intermediate conduit (45) between them.

6. The installation according to claim 1, **characterized in that** the gas sampling module (61) is arranged downstream of the NO injection device (24) and at a distance (D) comprised between 30 cm and 80 cm.

7. The installation according to claim 1, **characterized in that** the gas sampling module (61) is fluidically connected to the inspiratory branch (21) of the respiratory circuit (20).

8. The installation according to claim 4, **characterized in that** the gas sampling module (61) is fluidically connected to an upstream end (121) of the inspiratory branch (21).

9. The installation according to claims 1 and 4, **characterized in that** the respiratory circuit (20) comprises a junction piece (41) sealed to a downstream end (122) of the inspiratory branch (21), preferably the junction piece (41) is a Y-piece.

10. The installation according to claims 3 and 5, **characterized in that** the flow rate measurement device (25), the NO injection device (24), the gas sampling module (61), said at least one section of intermediate conduit (45) and the tubular connectors (46) form an independent and detachable connection assembly for connecting the NO delivery apparatus (1), configured to fluidically connect between the inspiratory branch (21) of the patient circuit (20) and the gas outlet (151) of the anesthesia ventilator (150).

11. The installation according to one of claims 1 or 6, **characterized in that** the gas sampling module (61) is arranged downstream of the NO injection device (24) and at a distance (D) of less than 60 cm.

12. The installation according to one of claims 1, 6 or 11, **characterized in that** the gas sampling module (61) is arranged downstream of the NO injection device (24) and at a distance (D) comprised between 30 cm and 50 cm.

13. The installation according to claim 1, **characterized in that** the gas sampling module (61) is fluidically connected to an inlet of the inspiratory branch (21) of the respiratory circuit (20), said respiratory circuit (20) being a rudimentary circuit comprising a junction piece (41) sealed located at a downstream end of the inspiratory branch (21).

14. The installation according to claim 1, **characterized in that**:
- the NO injection device (24) is fluidically connected to the NO delivery apparatus (1) so as to be supplied with gas containing NO by said NO delivery apparatus (1) and/or
- the flow rate measurement device (25) comprises a flow rate sensor fluidically connected to the NO delivery apparatus (1) so as to provide it with flow rate measurements, preferably the flow rate sensor is a mass flow rate sensor

15. The installation according to claim 1, **characterized in that** the gas sampling module (61) is fluidically connected to the NO delivery apparatus (1) so as to provide it with gas circulating in the inspiratory branch (21), downstream of the NO injection device (24).
